(19) Europäisches Patentamt / European Patent Office / Office européen des brevets

(11) **EP 3 485 806 A1**

(12) **EUROPEAN PATENT APPLICATION**

(43) Date of publication:
**22.05.2019 Bulletin 2019/21**

(21) Application number: **18184070.3**

(22) Date of filing: **17.07.2018**

(51) Int Cl.:
**A61B 5/0205** (2006.01)      **A61B 5/00** (2006.01)
**A61B 5/024** (2006.01)      A61B 5/0456 (2006.01)
A61B 5/0472 (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.11.2017   TW 106140055**

(71) Applicant: **Kinpo Electronics, Inc.
New Taipei City 22201 (TW)**

(72) Inventors:
 • **HARAIKAWA, Koichi**
   **22201 New Taipei City (TW)**
 • **CHIEN, Jen-Chien**
   **22201 New Taipei City (TW)**
 • **LIN, Yin-Tsong**
   **22201 New Taipei City (TW)**
 • **HUNG, Tsui-Shan**
   **22201 New Taipei City (TW)**
 • **HSIEH, Yi-Ta**
   **22201 New Taipei City (TW)**
 • **LIN, Chien-Hung**
   **22201 New Taipei City (TW)**

(74) Representative: **Emde, Eric
Wagner & Geyer
Gewürzmühlstrasse 5
80538 München (DE)**

(54) **WEARABLE DEVICE CAPABLE OF DETECTING SLEEP APNEA EVENT AND DETECTION METHOD THEREOF**

(57)      A wearable device capable of detecting sleep apnea comprising a processor and an electrocardiogram sensor is provided. The processor trains a neural network module to create a sleep apnea detection model. An electrocardiogram sensor senses an electrocardiogram signal of a sleep situation. The processor analyzes the electrocardiogram signal to detect a plurality of R-waves in the electrocardiogram signal. The processor performs an R-wave amplitude analysis operation, an R-wave angle analysis operation, and a heart rate variability analysis operation according to the R-waves to obtain a plurality of characteristic values. The processor utilizes the trained sleep apnea detection model to perform a sleep apnea detection operation based on the characteristic values, so as to detect whether the sleep situation has a sleep apnea event.

**EP 3 485 806 A1**

**Description**

Technical Field

The disclosure relates to a sleep detection technique, and more particularly, to a wearable device capable of detecting sleep apnea event and a detection method of sleep apnea event.

BACKGROUND

In the technical field for detecting sleep apnea event, an apnea hypopnea index (AHI) of a tester is generally evaluated by using a polysomnography (PSG) system. The apnea hypopnea index is used to evaluate a number of times that the tester shows a sleep apnea symptom in a sleep situation per hour, wherein the sleep apnea symptom refers to a situation where a sleep apnea event duration exists over 10 seconds per minute. However, in a common method for obtaining the apnea hypopnea index, a plurality of measurement information obtained from multiple physiological sensing operations performed by the PSG system are required so then the apnea hypopnea index can be obtained after going through complex calculations. However, the physiological sensing operations include, for example, electroencephalography (EEG), sleep gesture, heart rate, electrooculography (EOG), electrocardiogram (ECG), electromyography (EMG), respiratory Effort, air flow, blood pressure, blood oxygen saturation ($SaO_2$), etc. In other words, the common detection technique for sleep apnea event requires complicated accessories to be worn by a wearer and requires analysis on a large amount of sense data. In consideration of the above, providing a wearable device capable of effectively detecting whether the sleep situation of the wearer has sleep apnea event with characteristics of convenience is one of important issues to be addressed in the field.

SUMMARY

The disclosure is directed to a wearable device capable of detecting sleep apnea event and a detection method of sleep apnea event, which are capable of effectively detecting whether a sleep situation has a sleep apnea event.
The wearable device capable of detecting sleep apnea event of the disclosure includes a processor and an electrocardiogram sensor. The processor is configured to train a neural network module, so as to create a sleep apnea detection model. The electrocardiogram sensor is coupled to the processor. The electrocardiogram sensor is configured to sense an electrocardiogram signal of a sleep situation. The processor analyzes the electrocardiogram signal to detect a plurality of R-waves in the electrocardiogram signal. The processor performs an R-wave amplitude analysis operation, an R-wave angle analysis operation, and a heart rate variability analysis operation according to the R-waves, so as to obtain a plurality of first characteristic values, a plurality of second characteristic values and a plurality of third characteristic values. The processor utilizes the trained sleep apnea detection model to perform a sleep apnea detection operation based on the first characteristic values, the second characteristic values and the third characteristic values, so as to detect whether the sleep situation has a sleep apnea event.
The detection method of sleep apnea event of the disclosure is adapted to the wearable device capable of detecting sleep apnea event. The wearable device capable of detecting sleep apnea event includes a processor and an electrocardiogram sensor. The detection method of sleep apnea event includes steps of: training a neural network module by the processor to create a sleep apnea detection model; sensing an electrocardiogram signal of a sleep situation by the electrocardiogram sensor, and analyzing the electrocardiogram signal by the processor to detect a plurality of R-waves in the electrocardiogram signal, performing an R-wave amplitude analysis operation, an R-wave angle analysis operation, and a heart rate variability analysis operation according to the R-waves by the processor, so as to obtain a plurality of first characteristic values, a plurality of second characteristic values and a plurality of third characteristic values; and utilizing the trained sleep apnea detection model to perform a sleep apnea detection operation by the processor based on the first characteristic values, the second characteristic values and the third characteristic values, so as to detect whether the sleep situation has a sleep apnea event.
Based on the above, the wearable device capable of detecting sleep apnea event and a detection method of sleep apnea event of the disclosure are capable of effectively detecting whether the sleep situation has the sleep apnea event. The wearable device capable of detecting sleep apnea event of the disclosure can create the sleep apnea detection model in advance, and can effectively sense the electrocardiogram signal, so as to obtain the characteristic values of the electrocardiogram signal. Therefore, the processor of the wearable device capable of detecting sleep apnea event can input the characteristic values into the sleep apnea detection model for calculation, so as to effectively detect whether the sleep situation has the sleep apnea event.
To make the above features and advantages of the disclosure more comprehensible, several embodiments accompanied with drawings are described in detail as follows.

BRIEF DESCRIPTION OF THE DRAWINGS

The accompanying drawings are included to provide a further understanding of the disclosure, and are incorporated in and constitute a part of this specification. The drawings illustrate embodiments of the disclosure and, together with the description, serve to explain the principles of the disclosure.

FIG. 1 illustrates a schematic diagram of a wearable device in an embodiment of the disclosure.

FIG. 2 illustrates a schematic diagram of a plurality of modules and a detection model in an embodiment of the disclosure.

FIG. 3 illustrates a waveform diagram of an electrocardiogram signal in an embodiment of the disclosure.

FIG. 4 is a schematic diagram illustrating an R-wave angle analysis operation in an embodiment of the disclosure.

FIG. 5 is a schematic diagram illustrating an R-wave amplitude analysis operation in an embodiment of the disclosure.

FIG. 6 illustrates a flowchart of a detection method of sleep apnea event in an embodiment of the disclosure.

DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

In order to make content of the disclosure more comprehensible, embodiments are provided below to describe the disclosure in detail, however, the disclosure is not limited to the provided embodiments, and the provided embodiments can be suitably combined. Moreover, elements/components/steps with same reference numerals represent same or similar parts in the drawings and embodiments.

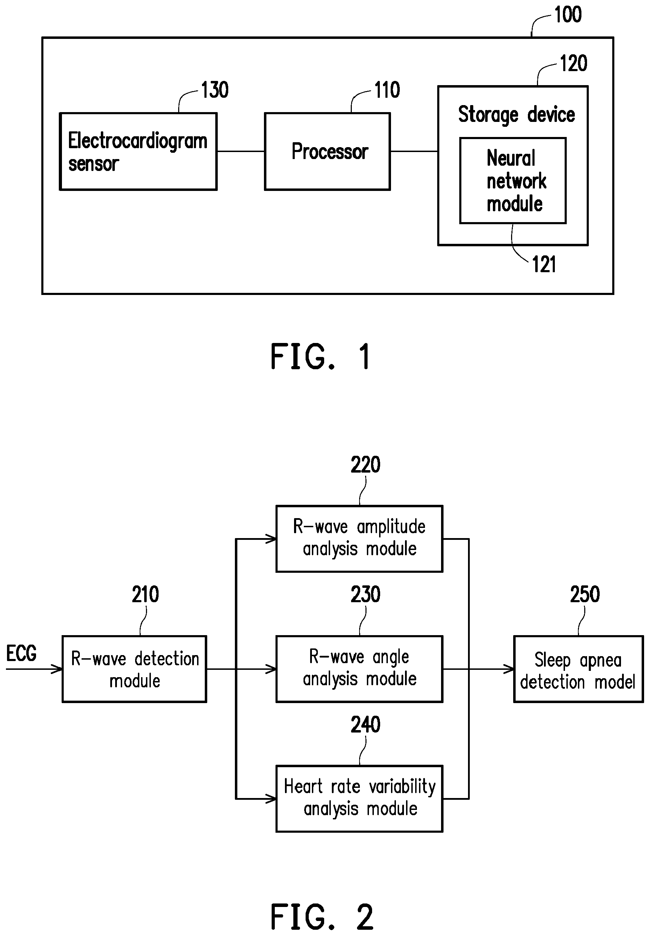

FIG. 1 illustrates a schematic diagram of a wearable device in an embodiment of the disclosure. With reference to FIG. 1, a wearable device 100 capable of detecting sleep apnea event includes a processor 110, a storage device 120 and an electrocardiogram sensor 130. The processor 110 is coupled to the storage device 120 and the electrocardiogram sensor 130. The storage device 20 stores a neural network module 121. In the present embodiment, the processor 110 trains a neural network module 121 to create a sleep apnea detection model. The electrocardiogram sensor 130 senses an electrocardiogram signal ECG, and the processor 110 analyzes the electrocardiogram signal to detect a plurality of R-waves in the electrocardiogram signal. In the present embodiment, the processor 110 performs an R-wave amplitude analysis operation, an R-wave angle (ECG Derived Respiratory Angle, EDRAG) analysis operation, and a heart rate variability (HRV) analysis operation according to the R-waves to obtain a plurality of characteristic values. The R-wave amplitude analysis operation is, for example, to analyze an R-wave amplitude EDR in the electrocardiogram in order to obtain an ECG-derived respiratory signal, wherein peak and trough of the R-wave may be used as the R-wave amplitude EDR. A difference between the peaks of two adjacent R-waves may be used as the adjacent R-waves difference.

In the present embodiment, the processor 110 utilizes the trained sleep apnea detection model to perform a sleep apnea detection operation based on the characteristic values, so as to detect whether the sleep situation has a sleep apnea event. In the present embodiment, the sleep apnea detection operation refers to an operation of inputting the characteristic values into the trained sleep apnea detection model for calculation performed by the processor 110 in order to obtain an apnea hypopnea index (AHI). Accordingly, the wearable device 100 of the present embodiment can determine whether the sleep situation of the wearer has the sleep apnea event according to the apnea hypopnea index.

In the present embodiment, the wearable device 100 may be, for example, smart clothes, smart wristbands or other similar devices, and the wearable device 100 is configured to detect the sleep situation of the wearer. The wearable device 100 can integrate each of said circuit elements into one wearable item instead of complicated accessories. In other words, when the wearer is asleep, whether the sleep situation of the wearer has the sleep apnea event may be detected by the wearable device 100 worn by the wearer. In the present embodiment, the processor 110 uses an Apnea-ECG database in a Physionet data platform as a training target to train the neural network module 121 in advance based on another plurality of characteristic values from a plurality of sleep situation samples, so as create the sleep apnea detection model.

In the present embodiment, the processor 110 is, for example, a central processing unit (CPU), a system on chi (SOC) or other programmable devices for general purpose or special purpose, such as a microprocessor and a digital signal processor (DSP), a programmable controller, an application specific integrated circuit (ASIC), a programmable logic device (PLD) or other similar devices or a combination of above-mentioned devices.

In the present embodiment, the storage device 120 is, for example, a dynamic random access memory (DRAM), a flash memory or a non-volatile random access memory (NVRAM). In the present embodiment, the storage device 120 is configured to store data and program modules described in each embodiment of the disclosure, which can be read and executed by the processor 110 so the wearable device 100 can realize the detection method of sleep apnea event described in each embodiment of the disclosure.

In the present embodiment, the storage device 120 is stored with the neural network module 121. The processor 110 can sense a plurality of characteristic values from different wearers in advance by the electrocardiogram sensor 130, and use the characteristic values from the different wearers as sample data. In the present embodiment, the processor 110 can create a prediction model according to determination conditions, algorithms and parameters from the sleep stage, and use a plurality of the sample data for training or correcting the prediction model. Accordingly, when the sleep apnea detection operation is performed by the wearable device 100 for the wearer, the processor 110 can utilize the trained sleep apnea detection model to obtain a detection result according to the characteristic values sensed by the electrocardiogram sensor 130. Nevertheless, enough teaching, suggestion, and implementation illustration regarding algorithms and calculation modes for the trained neural network module 121 of the present embodiment may be obtained with reference to common knowledge in the related art, which is not repeated hereinafter.

FIG. 2 illustrates a schematic diagram of a plurality of modules and a detection model in an embodiment of the disclosure. With reference to FIG. 1 and FIG. 2, in the present embodiment, the processor 110 can execute an R-wave detection module 210, an R-wave amplitude analysis module 220, an R-wave angle analysis module 230, a heart rate variation analysis module 240 and a sleep apnea detection model 250 as shown in FIG. 2. In the present embodiment, the electrocardiogram sensor 130 senses an electrocardiogram sensor ECG of the wearer. The wearable device 100 takes a signal length of one minute spaced by 30 seconds as a segment, for example, and performs the sleep apnea detection operation on the segment. In the present embodiment, the processor 110 receives the electrocardiogram signal ECG, and the processor 110 executes the R-wave detection module 210 to analyze the electrocardiogram signal ECG. In the present embodiment, the processor 110 executes the R-wave amplitude analysis module 220, the R-wave angle analysis module 230 and the heart rate variation analysis module 240 to analyze the R-waves of the electrocardiogram signal ECG.

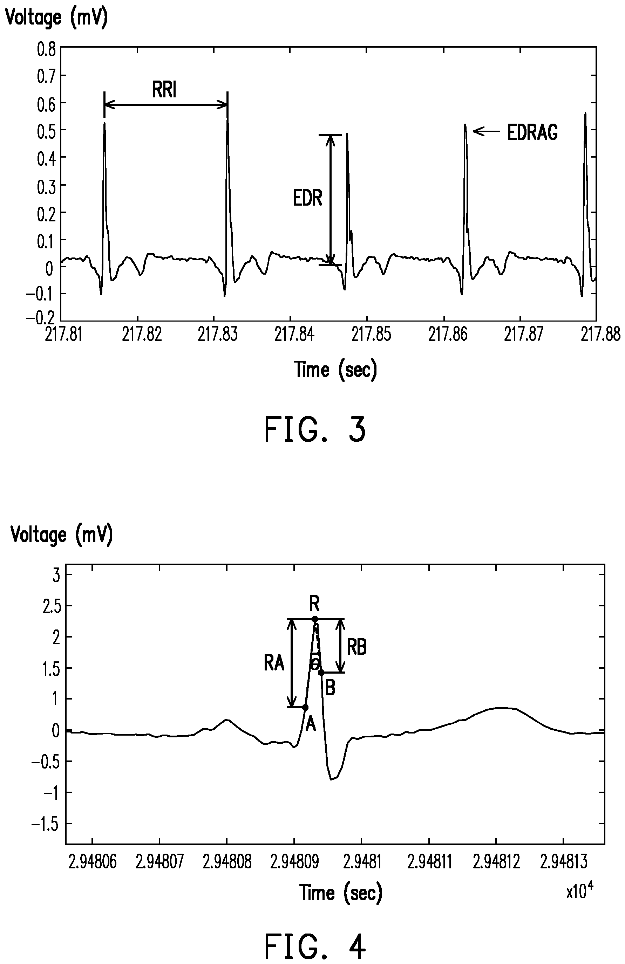

FIG. 3 illustrates a waveform diagram of an electrocardiogram signal in an embodiment of the disclosure. With reference to FIG. 1 to FIG. 3, the processor 110 executes the R-wave amplitude analysis module 220 to perform the R-wave amplitude analysis operation so the processor 110 can analyze amplitudes of R-wave signals. The processor 110 executes the R-wave angle analysis module 230 to perform the R-wave angle analysis operation so the processor 110 can analyze angles of the amplitudes of the R-wave signals. The processor 110 executes the heart rate variability analysis module 240 to perform the heart rate variability analysis operation so the processor 110 can analyze heart beat intervals of the electrocardiogram signal ECG to obtain a heart beat intervals variation RRI among the R-wave signals in the electrocardiogram signal ECG. For instance, in the present embodiment, a distance between two R-waves may be used as the heart beat interval variation RRI. A distance between a peak and a trough of the R-wave may be used as the R-wave amplitude EDR. Also, an included angle provided at the peak of the R-wave amplitude EDR may be used as an R-wave angle EDRAG.

In the present embodiment, the processor 110 executes the heart rate variability analysis module 240 to perform the heart rate variability analysis operation, so as to analyze a heart beat intervals variation RRI of the electrocardiogram signal ECG. The processor 110 calculates time intervals among adjacent R-waves within the segment of the electrocardiogram signal ECG to generate the heart beat intervals variation RRI signal. In the present embodiment, the processor 110 calculates a plurality of characteristic values according to the heart beat intervals variation RRI signal. In other words, the wearable device 100 capable of detecting sleep apnea event of the present embodiment can take into consideration of an effect of sympathetic nerve and parasympathetic nerve of the wearer on respiration.

In the present embodiment, the characteristic values obtained by the processor 110 include a heart beat interval mean, a heart rate (HR) value, a heart beat interval standard deviation (Standard Deviation of Normal to Normal; SDNN), a number of adjacent R-waves differences over 50 ms (Number of pairs of adjacent NN intervals differing by more than 50 ms in the entire recording; NN50), a ratio of the adjacent R-waves differences over 50 ms (NN50 count divided by the total number of all NN intervals), a root mean square of the adjacent R-waves differences (Root Mean Square of the Successive Differences; RMSSD), a vertical axis standard deviation (SD1), a horizontal axis standard deviation (SD2), and a first ratio (SD ratio) of the vertical axis standard deviation and the horizontal axis standard deviation in a Poincare plot, a low frequency range power (LFP), a high frequency range power, a total power (TP), a very low frequency range power (VLFP), a normalize low frequency power (nLFP), a normalize high frequency power (nHFP), and a second ratio (LF/HF) between a high frequency (HF) and a low frequency (LF). In other words, in the present embodiment, the processor 110 can execute the heart rate variability analysis module 240 to obtain the 16 characteristic values described above.

FIG. 4 is a schematic diagram illustrating an R-wave angle analysis operation in an embodiment of the disclosure. With reference to FIG. 1 to FIG. 4, in the present embodiment, the processor 110 uses the R-wave angle EDRAG as an effect of abdominal breathing of the wearer on the electrocardiogram signal ECG. Specifically, the processor 110 uses a peak R of the R-wave amplitude EDR as a center point to select positions of voltage values which are 0.01 second before and after the center point on an R-wave curve in the electrocardiogram signal ECG as a reference point A and a reference point B, respectively. The processor 110 calculates an arctangent value according to a distance RA between the reference point A and the peak R and a duration of 0.01 second, and the processor 110 calculate another arctangent value according to a distance RB between the reference point B and the peak R and a duration of 0.01 second. Also, the processor 110 calculates the two arctangent values by a trigonometric function to obtain an included angle θ between a line from the reference point A to the peak R and another line from the reference point B to the peak R. In this way, in the present embodiment, the processor 110 can perform the above calculation for each of the R-waves within the signal length of one minute to obtain the R-wave angle EDRAG signals. The specific calculation may be, for example, Formula (1) provided below.

$$\tan^{-1}\left(\frac{0.01}{RA}\right) + \tan^{-1}\left(\frac{0.01}{RB}\right) = \theta \ldots\ldots\ldots\ldots \text{Formula (1)}$$

In the present embodiment, the processor 110 further calculates the R-wave angle EDRAG signal to obtain an R-wave angle mean, an R-wave angle standard deviation, an R-wave angle sample entropy (SE), and an R-wave angle detrended fluctuation analysis value (DFA). Further, the processor 110 estimates energy distributions of the R-wave angle EDRAG signal spaced by the 0.3Hz within a frequency domain of 0 to 6Hz. Here, 20 frequency domain intervals are provided such 20 characteristic values may be obtained. In other words, in the present embodiment, the processor 110 can execute R-wave angle analysis module 230 to generate the 24 characteristic values described above.

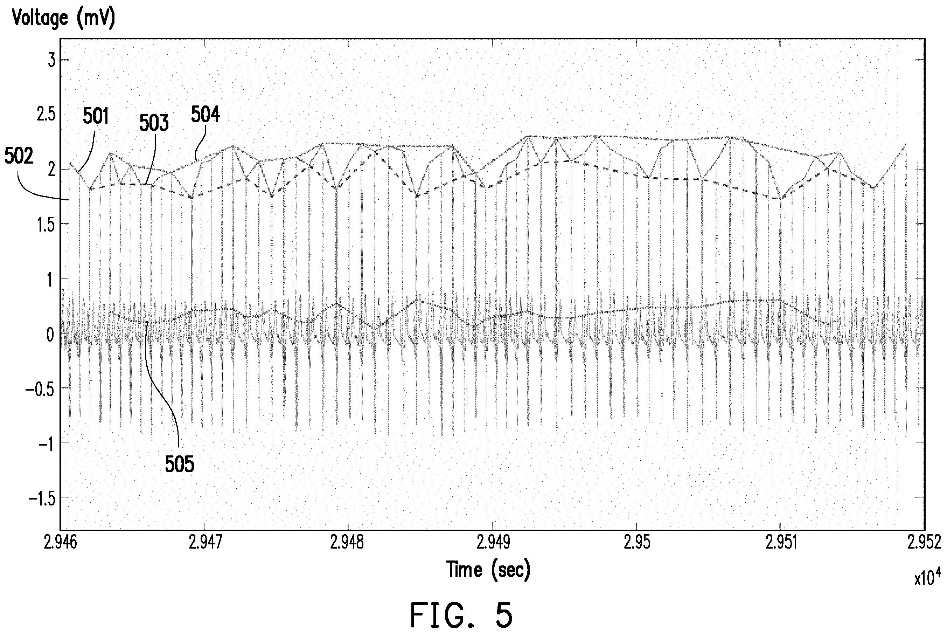

FIG. 5 is a schematic diagram illustrating an R-wave amplitude analysis operation in an embodiment of the disclosure. With reference to FIG. 1 to FIG. 3 and FIG. 5, in the present embodiment, the processor 110 samples a plurality of R-wave signals 502 of the electrocardiogram signal ECG, and connects positions of the R-wave signals 502 together to obtain an R-wave amplitude EDR signal 501. The processor 110 obtains an EDR peak wave packet signal 504 and an EDR bottom wave packet signal 503 according to the R-wave amplitude EDR signal 501. The processor 110 subtracts the EDR bottom wave packet signal 503 from the EDR peak wave packet signal 504 to obtain an EDR wave packets-subtracted signal 505. In the present embodiment, the processor 110 calculates an R-wave amplitude mean, an R-wave amplitude standard deviation, an R-wave amplitude sample entropy, and an R-wave amplitude detrended fluctuation analysis value of the segment of the EDR wave packets-subtracted signal 505. Moreover, in the present embodiment, the processor estimates power distributions of the R-wave amplitude EDR signal 501 within a frequency domain of 0 to 6Hz through a fast Fourier transform, which are spaced by 0.3Hz. Here, 20 frequency domain intervals are provided such that a plurality of R-wave amplitude power distributions of the R-wave amplitude signal may be estimated. In other words, in the present embodiment, the processor 110 can execute R-wave amplitude analysis module 220 to generate the 24 characteristic values described above.

Referring back to FIG. 1 and FIG. 2, in other words, the processor 110 of the wearable device 100 of the present embodiment can input the 64 specific characteristic values described in the foregoing embodiments from FIG. 3 to FIG. 5 into the trained sleep apnea detection model 250. The sleep apnea detection model 250 has a neural network architecture. The processor 110 uses said 64 characteristic values as input parameters, and obtain at least one output parameter through weights and effect parameters in the neural network architecture. Said at least one output parameter may be, for example, the apnea hypopnea index. In this way, the wearable device 100 of the present embodiment can provide the function of effectively detecting whether the sleep situation has the sleep apnea event. Nevertheless, enough teaching, suggestion, and implementation illustration regarding related signal analysis and parameter calculation described in the embodiments of the disclosure may be obtained with reference to common knowledge in the related art of electrocardiogram, which is not repeated hereinafter.

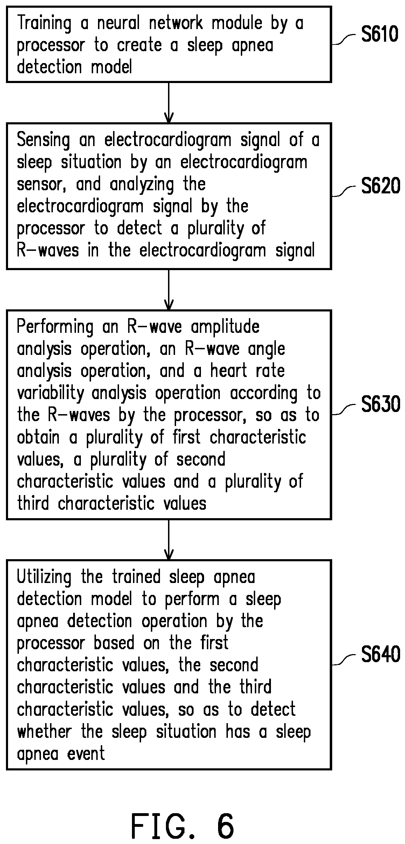

FIG. 6 illustrates a flowchart of a detection method of sleep apnea event in an embodiment of the disclosure. With reference to FIG. 1 and FIG. 6, the detection method of sleep apnea event of the present embodiment is at least adapted to the wearable device 100 of FIG. 1. In step S610, the processor 110 trains a neural network module 121 to create a sleep apnea detection model. In step S620, the electrocardiogram sensor 130 senses an electrocardiogram signal of a sleep situation, and the processor 110 analyzes the electrocardiogram signal to detect a plurality of R-waves in the electrocardiogram signal. In step S630, the processor 110 performs an R-wave amplitude analysis operation, an R-wave angle analysis operation, and a heart rate variability analysis operation according to the R-waves, so as to obtain a plurality of first characteristic values, a plurality of second characteristic values and a plurality of third characteristic values. In step S640, the processor 110 utilizes the trained sleep apnea detection model to perform a sleep apnea detection operation based on the first characteristic values, the second characteristic values and the third characteristic values, so as to detect whether the sleep situation has a sleep apnea event.

In the present embodiment, the first characteristic values refer to the 24 characteristic values generated by the R-wave amplitude analysis operation described in the embodiment of FIG. 5. The second characteristic values refer to the 24 characteristic values generated by the R-wave angle analysis operation described in the embodiment of FIG. 4. The third characteristic values refer to the 16 characteristic values generated by the heart rate variability analysis operation described in the embodiment of FIG. 3. Further, the processor 110 can input said 64 characteristic values into the trained sleep apnea detection model in order to obtain an apnea hypopnea index. In this way, the detection method of sleep apnea event of the present embodiment is capable of effectively detecting whether the sleep situation has the sleep apnea event.

In addition, sufficient teaching, suggestion, and implementation regarding technical details of each of relevant elements or analysis operations of the wearable device 100 of the present embodiment of the disclosure may be obtained from the foregoing embodiments of FIG. 1 to FIG. 5, and thus related descriptions thereof are not repeated hereinafter.

In summary, the wearable device capable of detecting sleep apnea event and the detection method of sleep apnea event of the disclosure can create the sleep apnea detection model in advance, and can effectively sense the electrocardiogram signal in the sleep situation so as to obtain 24 specific characteristic values of the electrocardiogram signal. Also, the processor of the wearable device of the disclosure can input the 64 specific characteristic values into the sleep apnea detection model for calculation in order to effectively obtain the apnea hypopnea index of the wearer. Accordingly, the wearable device of the disclosure can effectively and accurately determine whether the sleep situation of the wearer has the sleep apnea event according to the apnea hypopnea index. Furthermore, only the electrocardiogram sensor is required to be disposed in the wearable device of the disclosure. As a result, the wearable device of the disclosure can be made without complicated accessories to provide characteristics of convenience.

**Claims**

1. A wearable device (100) capable of detecting sleep apnea event, comprising:

   a processor (110), configured to train a neural network module (121), so as to create a sleep apnea detection model; and
   an electrocardiogram sensor (130), coupled to the processor (110), and configured to sense an electrocardiogram signal (ECG) of a sleep situation, the processor (110) analyzing the electrocardiogram signal (ECG) to detect a plurality of R-waves (502) in the electrocardiogram signal (ECG),
   wherein the processor (110) performs an R-wave amplitude analysis operation, an R-wave angle analysis operation, and a heart rate variability analysis operation according to the R-waves (502), so as to obtain a plurality of first characteristic values, a plurality of second characteristic values and a plurality of third characteristic values,
   wherein the processor (110) utilizes the trained sleep apnea detection model to perform a sleep apnea detection operation based on the first characteristic values, the second characteristic values and the third characteristic values, so as to detect whether the sleep situation has a sleep apnea event.

2. The wearable device (100) according to claim 1, wherein the processor (110) performs the R-wave amplitude analysis operation according to the R-waves (502) to obtain an R-wave amplitude signal (501),
   wherein the processor (110) analyzes the R-wave amplitude signal (501) to obtain an R-wave amplitude mean, an R-wave amplitude standard deviation, an R-wave amplitude sample entropy, and an R-wave amplitude detrended fluctuation analysis value, and the processor (110) estimates the R-wave amplitude signal (501) through a fast Fourier transform, so as to estimate a plurality of R-wave amplitude power distributions of the R-wave amplitude signal (501),
   wherein the first characteristic values comprise the R-wave amplitude mean, the R-wave amplitude standard deviation, the R-wave amplitude sample entropy, the R-wave amplitude detrended fluctuation analysis value and the R-wave amplitude power distributions.

3. The wearable device (100) according to claim 2, wherein the processor (110) analyzes the R-wave amplitude signal (501) to obtain a peak wave packet signal (504) and a bottom wave packet signal (503) of the R-wave amplitude signal (501), and the processor (110) subtracts the bottom wave packet signal (503) from the peak wave packet signal (504) to obtain a wave packets-subtracted signal (505),
   wherein the processor (110) calculates the first characteristic values according to the wave packets-subtracted signal (505).

4. The wearable device (100) according to claim 2, wherein the processor (110) estimates the R-wave amplitude signal (501) through the fast Fourier transform, so as to estimate 20 R-wave amplitude power distributions of the R-wave amplitude signal (501) spaced by 0.3Hz within a frequency domain from 0 to 6Hz.

5. The wearable device (100) according to any one of claims 1 to 4, wherein the processor (110) performs the R-wave angle analysis operation according to the R-waves (502) to obtain an R-wave angle signal,
wherein the processor (110) analyzes the R-wave angle signal to obtain an R-wave angle mean, an R-wave angle standard deviation, an R-wave angle sample entropy, and an R-wave angle detrended fluctuation analysis value, and the processor (110) estimates the R-wave angle signal through a fast Fourier transform, so as to estimate a plurality of R-wave angle power distributions of the R-wave angle signal,
wherein the second characteristic values comprise the R-wave angle mean, the R-wave angle standard deviation, the R-wave angle sample entropy, the R-wave angle detrended fluctuation analysis value and the R-wave angle power distributions.

6. The wearable device (100) according to claim 5, wherein the processor (110) analyzes the R-waves (502) to obtain an R-wave peak position of each of the R-waves and two respective reference points (A, B) which are 0.01 second before and after the R-wave peak position, such that the processor (110) calculates a plurality of included angles of the R-waves (502) according to the R-wave peak position and the two respective reference points (A, B) of each of the R-waves (502).

7. The wearable device (100) according to claim 5, wherein the processor (110) estimates the R-wave angle signal through the fast Fourier transform, so as to estimate 20 R-wave angle power distributions of the R-wave angle signal spaced by 0.3Hz within a frequency domain from 0 to 6Hz.

8. The wearable device (100) according to any one of claims 1 to 7, wherein the processor (110) performs the heart rate variability analysis operation according to the R-waves (502) to analyze a heart beat intervals variation (RRI) of the electrocardiogram signal (ECG), so as to obtain the third characteristic values comprising a heart beat interval mean, a heart rate value, a heart beat interval standard deviation, a number of adjacent R-waves differences over 50 ms, a ratio of the adjacent
R-waves differences over 50 ms, a root mean square of the adjacent R-waves differences, a vertical axis standard deviation, a horizontal axis standard deviation, and a first ratio of the vertical axis standard deviation and the horizontal axis standard deviation in a Poincare plot, a low frequency range power, a high frequency range power, a total power, a very low frequency range power, a normalize low frequency power, a normalize high frequency power, and a second ratio between a high frequency and a low frequency.

9. A detection method of sleep apnea event, adapted to a wearable device (100) capable of detecting sleep apnea event, the wearable device (100) comprising a processor (110) and an electrocardiogram sensor (130), the method comprising:

training a neural network module (121) by the processor (110) to create a sleep apnea detection model;
sensing an electrocardiogram signal (ECG) of a sleep situation by the electrocardiogram sensor (130), and analyzing the electrocardiogram signal (ECG) by the processor (110) to detect a plurality of R-waves (502) in the electrocardiogram signal (ECG),
performing an R-wave amplitude analysis operation, an R-wave angle analysis operation, and a heart rate variability analysis operation according to the R-waves (502) by the processor (110), so as to obtain a plurality of first characteristic values, a plurality of second characteristic values and a plurality of third characteristic values; and
utilizing the trained sleep apnea detection model to perform a sleep apnea detection operation by the processor (110) based on the first characteristic values, the second characteristic values and the third characteristic values, so as to detect whether the sleep situation has a sleep apnea event.

10. The detection method of sleep apnea event according to claim 9, wherein the step of performing the R-wave amplitude analysis operation according to the R-waves (502) by the processor (110) to obtain the first characteristic values comprises:

performing the R-wave amplitude analysis operation according to the R-waves (502) by the processor (110) to obtain an R-wave amplitude signal (501);
analyzing the R-wave amplitude signal (501) by the processor (110) to obtain an R-wave amplitude mean, an R-wave amplitude standard deviation, an R-wave amplitude sample entropy, and an R-wave amplitude detrended fluctuation analysis value; and
estimating the R-wave amplitude signal (501) through a fast Fourier transform by the processor (110), so as to estimate a plurality of R-wave amplitude power distributions of the R-wave amplitude signal (501),
wherein the first characteristic values comprise the R-wave amplitude mean, the R-wave amplitude standard deviation, the R-wave amplitude sample entropy, the R-wave amplitude detrended fluctuation analysis value and the R-wave amplitude power distributions.

11. The detection method of sleep apnea event according to claim 10, wherein the step of performing the R-wave amplitude analysis operation according to the R-waves (502) by the processor (110) to obtain the first characteristic values comprises:

analyzing the R-wave amplitude signal (501) by the processor (110) to obtain a peak wave packet signal (504) and a bottom wave packet signal (503) of the R-wave amplitude signal (501), and subtracting the bottom wave packet signal (503) from the peak wave packet signal (504) by the processor (110) to obtain a wave packets-subtracted signal (505),
calculating the first characteristic values according to the wave packets-subtracted signal (505) by the processor (110).

12. The detection method of sleep apnea event according to any one of claims 9 to 11, wherein the step of performing the R-wave angle analysis operation according to the R-waves (502) by the processor (110) to obtain the second characteristic values comprises:

performing the R-wave angle analysis operation according to the R-waves (502) by the processor (110) to obtain an R-wave angle signal,
analyzing the R-wave angle signal by the processor (110) to obtain an R-wave angle mean, an R-wave angle standard deviation, an R-wave angle sample entropy, and an R-wave angle detrended fluctuation analysis value; and
estimating the R-wave angle signal through a fast Fourier transform by the processor (110), so as to estimate a plurality of R-wave angle power distributions of the R-wave angle signal,
wherein the second characteristic values comprise the R-wave angle mean, the R-wave angle standard deviation, the R-wave angle sample entropy, the R-wave angle detrended fluctuation analysis value and the R-wave angle power distributions.

13. The detection method of sleep apnea event according to claim 12, wherein the step of performing the R-wave angle analysis operation according to the R-waves (502) by the processor (110) to obtain the R-wave angle signal comprises:

analyzing the R-waves by the processor (110) to obtain an R-wave peak position of each of the R-waves (502) and two respective reference points (A, B) which are 0.01 second before and after the R-wave peak position, such that the processor (110) calculates a plurality of included angles of the R-waves according to the R-wave peak position and the two respective reference points (A, B) of each of the R-waves.

14. The detection method of sleep apnea event according to claim 10 or 12, wherein the processor (110) estimates the R-wave amplitude signal (501) or the R-wave angle signal through the fast Fourier transform, so as to estimate 20 R-wave amplitude power distributions of the R-wave amplitude signal (501) spaced by 0.3Hz within a frequency domain from 0 to 6Hz, or estimate 20 R-wave angle power distributions of the R-wave angle signal spaced by 0.3Hz within a frequency domain from 0 to 6Hz.

15. The detection method of sleep apnea event according to any one of claims 9 to 14, wherein the step of performing the heart rate variability analysis operation according to the R-waves (502) by the processor (110) to obtain the third characteristic values comprises:

performing the heart rate variability analysis operation according to the R-waves (502) by the processor (110) to analyze a heart beat intervals variation (RRI) of the electrocardiogram signal (ECG), so as to obtain the third characteristic values comprising a heart beat interval mean, a heart rate value, a heart beat interval standard deviation, a number of adjacent R-waves differences over 50 ms, a ratio of the adjacent R-waves differences over 50 ms, a root mean square of the adjacent R-waves differences, a vertical axis standard deviation, a horizontal axis standard deviation, and a first ratio of the vertical axis standard deviation and the horizontal axis standard deviation in a Poincare plot, a low frequency range power, a high frequency range power, a total power, a very low frequency range power, a normalize low frequency power, a normalize high frequency power, and a second ratio between a high frequency and a low frequency.

100

120

130

110

Storage device

Electrocardiogram
sensor

Processor

Neural
network
module

121

## FIG. 1

220

R—wave amplitude
analysis module

210

230

250

ECG

R—wave detection
module

R—wave angle
analysis module

Sleep apnea
detection model

240

Heart rate variability
analysis module

## FIG. 2

FIG. 3

FIG. 4

FIG. 5

Training a neural network module by a processor to create a sleep apnea detection model — S610

Sensing an electrocardiogram signal of a sleep situation by an electrocardiogram sensor, and analyzing the electrocardiogram signal by the processor to detect a plurality of R-waves in the electrocardiogram signal — S620

Performing an R-wave amplitude analysis operation, an R-wave angle analysis operation, and a heart rate variability analysis operation according to the R-waves by the processor, so as to obtain a plurality of first characteristic values, a plurality of second characteristic values and a plurality of third characteristic values — S630

Utilizing the trained sleep apnea detection model to perform a sleep apnea detection operation by the processor based on the first characteristic values, the second characteristic values and the third characteristic values, so as to detect whether the sleep situation has a sleep apnea event — S640

FIG. 6

Europäisches
Patentamt

European
Patent Office

Office européen
des brevets

**EUROPEAN SEARCH REPORT**

Application Number

EP 18 18 4070

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/039999 A1 (UNIV OKLAHOMA STATE [US]) 13 March 2014 (2014-03-13)<br>* page 4, lines 21, 22 *<br>* page 2, line 27 *<br>* page 11, line 5 *<br>* page 15, lines 4-7, 10, 12, 13 *<br>* page 16, lines 2, 3, 7-10, 14, 17, 18 *<br>* page 18, lines 4, 14 *<br>* tables 3, 4 *<br>----- | 1-15 | INV.<br>A61B5/0205<br>A61B5/00<br>A61B5/024<br><br>ADD.<br>A61B5/0456<br>A61B5/0472 |
| A | AYYOOB JAFARI: "Sleep apnoea detection from ECG using features extracted from reconstructed phase space and frequency domain",<br>BIOMEDICAL SIGNAL PROCESSING AND CONTROL, vol. 8, no. 6, 19 June 2013 (2013-06-19), pages 551-558, XP055554648,<br>NL<br>ISSN: 1746-8094, DOI: 10.1016/j.bspc.2013.05.007<br>* page 551, right-hand column, lines 8, 9 *<br>* page 552, left-hand column, lines 25-27 *<br>----- | 2,10 | |
| A | P. DE CHAZAL ET AL: "Automatic classification of sleep apnea epochs using the electrocardiogram",<br>COMPUTERS IN CARDIOLOGY 2000 CAMBRIDGE, MA, USA 24-27 SEPT. 2000,<br>1 January 2000 (2000-01-01), pages 745-748, XP055554559,<br>US<br>DOI: 10.1109/CIC.2000.898632<br>ISBN: 978-0-7803-6557-5<br>* page 746, right-hand column, line 10 *<br>* page 747, left-hand column, lines 2-5 *<br>----- | 2,3,7,10,11 | |

TECHNICAL FIELDS SEARCHED (IPC)

A61B

-/--

The present search report has been drawn up for all claims

| Place of search | Date of completion of the search | Examiner |
|---|---|---|
| The Hague | 12 February 2019 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 18 18 4070

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2005/267362 A1 (MIETUS JOSEPH E [US] ET AL) 1 December 2005 (2005-12-01) * paragraph [0044] - paragraph [0053] * | 4,14 | |
| A | ESTHER PUEYO ET AL: "QRS Slopes for Detection and Characterization of Myocardial Ischemia", IEEE TRANSACTIONS ON BIOMEDICAL ENGINEERING, IEEE SERVICE CENTER, PISCATAWAY, NJ, USA, vol. 55, no. 2, 1 February 2008 (2008-02-01), pages 468-477, XP011200289, ISSN: 0018-9294, DOI: 10.1109/TBME.2007.902228 * page 469, right-hand column, lines 20-47 * * figure 1 * | 6,13 | |
| A | US 2016/367157 A1 (BLAKE MICHAEL [US] ET AL) 22 December 2016 (2016-12-22) * figure 2 * | 8,15 | TECHNICAL FIELDS SEARCHED (IPC) |

The present search report has been drawn up for all claims

| Place of search | Date of completion of the search | Examiner |
|---|---|---|
| The Hague | 12 February 2019 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 18 4070

This annex lists the patent family members relating to the patent documents cited in the above-mentioned European search report.
The members are as contained in the European Patent Office EDP file on
The European Patent Office is in no way liable for these particulars which are merely given for the purpose of information.

12-02-2019

| Patent document cited in search report | | Publication date | Patent family member(s) | | Publication date |
|---|---|---|---|---|---|
| WO 2014039999 | A1 | 13-03-2014 | US 2014107501 A1 | | 17-04-2014 |
| | | | WO 2014039999 A1 | | 13-03-2014 |
| US 2005267362 | A1 | 01-12-2005 | AU 2006269263 A1 | | 18-01-2007 |
| | | | CA 2619617 A1 | | 18-01-2007 |
| | | | DK 2526858 T3 | | 10-06-2014 |
| | | | EP 1906817 A2 | | 09-04-2008 |
| | | | EP 2526858 A1 | | 28-11-2012 |
| | | | ES 2464151 T3 | | 30-05-2014 |
| | | | JP 5005687 B2 | | 22-08-2012 |
| | | | JP 2009501060 A | | 15-01-2009 |
| | | | US 2005267362 A1 | | 01-12-2005 |
| | | | US 2010069762 A1 | | 18-03-2010 |
| | | | WO 2007008706 A2 | | 18-01-2007 |
| US 2016367157 | A1 | 22-12-2016 | NONE | | |

EPO FORM P0459

For more details about this annex : see Official Journal of the European Patent Office, No. 12/82